(19) Europäisches Patentamt
European Patent Office
Office européen des brevets

(11) Publication number: **0 313 919 A2**

(12) **EUROPEAN PATENT APPLICATION**

(21) Application number: 88116906.4

(51) Int. Cl.⁴: **C07D 237/26 , G01N 33/533**

(22) Date of filing: 12.10.88

(30) Priority: **28.10.87 US 114316**

(43) Date of publication of application: **03.05.89 Bulletin 89/18**

(84) Designated Contracting States: **AT BE CH DE ES FR GB IT LI NL**

(71) Applicant: **ABBOTT LABORATORIES**

**Abbott Park Illinois 60064(US)**

(72) Inventor: **Chan, Clifford Man**
**17652 West Windslow Drive**
**Grayslake Illinois 60030(US)**

(74) Representative: **Modiano, Guido et al**
**MODIANO, JOSIF, PISANTY & STAUB**
**Modiano & Associati Baaderstrasse 3**
**D-8000 München 5(DE)**

(54) **A novel synthesis of chemiluminescence labels for immuno assays.**

(57) Cyclic hydrazides and methods for synthesizing them are disclosed. Specifically, chemiluminescent phenanthrene cyclic hydrazides may be manufactured by a process employing modified Diels-Alder reaction.

A cyclic hydrazide of a cyclic compound may be synthesized by adding an α,Ω-acetylenedicarboxylic acid ester to an ethylene substituent of a cyclic compound to form a cyclic dicarboxylate of the cyclic compound, and reacting the cyclic dicarboxylate with hydrazine to form a chemiluminescent cyclic hydrazide.

Phenanthrene cyclic hydrazides according to the present invention include 9-[N-ethyl-N-butyl] aminophenanthrene-1,2-dicarboxylic acid hydrazide and 9-(dipropane sulfonic acid)aminophenanthrene-1,2-dicarboxylic acid hydrazide.

FIG.2

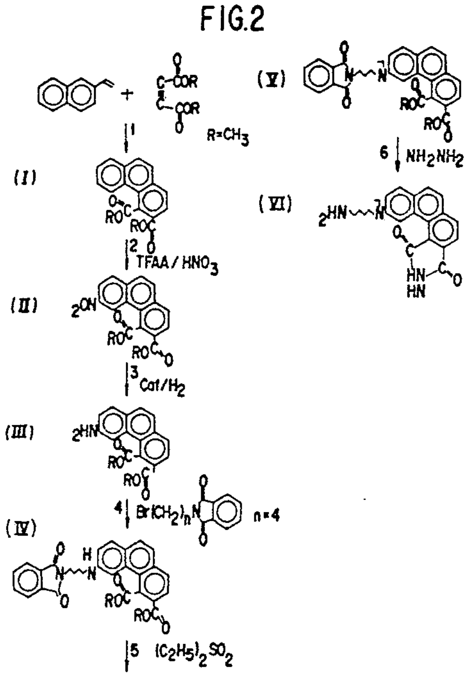


EP 0 313 919 A2

## A NOVEL SYNTHESIS OF CHEMILUMINESCENCE LABELS FOR IMMUNO ASSAYS

### Background Of The Invention

The present invention pertains in general to cyclic hydrazides and to methods for synthesizing them. Specifically, the present invention pertains to chemiluminescent phenanthrene cyclic hydrazides and methods for their manufacture.

Chemiluminescence may be defined as the generation of light from a chemical reaction. The mechanism of most chemiluminescent reactions is not known in detail, but a generalized mechanism [Schuster et al., Advances in Physical Organic Chemistry, 187-238 (1984)] may be outlined:

$A \rightarrow B^{*} \rightarrow B + h\nu$

Compound A undergoes a chemical reaction (usually oxidation) to yield a product in an electronically excited State ("$B^{*}$"). As it returns to the ground state ("B"), this product gives up energy in the form of light ("$h\nu$").

Chemiluminescence has been used for a variety of purposes in analytical chemistry where other methods are not sufficiently sensitive. In immunodiagnostics, chemiluminescent immunoassays ("CLIA") may match or exceed the sensitivity of radioimmunoassays ("RIA") or enzyme immunoassays ("EIA") [Kircka et al., Diagnostic Medicine, 1, 45-52 (1984)].

Many cyclic hydrazides are chemiluminescent. Examples of chemiluminescent cyclic hydrazides include luminol, isoluminol and their derivatives. Other examples are naphthalene-1-2 dicarboxylic hydrazide, and 7-amino-butylethyl naphthalene 1,2 dicarboxylic hydrazide.

Schroeder et al., U.K. Patent Application GB 2,026,158, and Buckler et al., U.S. Patent No. 4,225,485, disclose chemiluminescent napthalene-1,2-dicarboxylic acid hydrazides and synthesis thereof employing a dimethyl 7-aminonaphthalene-1,2 dicarboxylate obtainable according to the procedure of Gundermann et al., Justus Liebigs Ann. Chem., 10, 1873-1888 (1976). The disclosed compounds are used as labels in specific binding assays for detecting a ligand or a binding partner thereof.

Wagner-Jauregg, Synthesis, 1980, 769-798 (1980) and Diment et al., Aust. J. Chem., 22, 1721-30 (l969) disclose reaction of the natural coumarin derivatives xanthyletin and xanthoxyletin with dimethyl acetylenedicarboxylate to form a benzocoumarin derivative as follows:

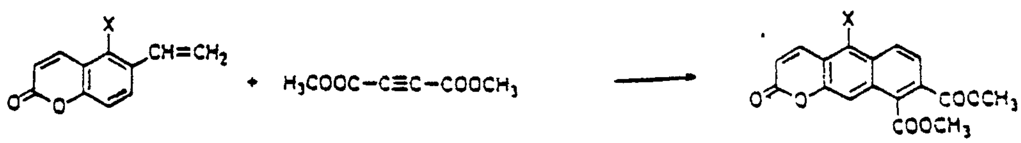

wherein X is H for xanthyletin, and wherein X is $OCH_3$ for xanthoxyletin. The reaction is described as a variation of the Diels-Alder reaction.

Wagner-Jauregg, Synthesis, 1980, 769-798 (1980) and Acheson, J. Chem. Soc. (C), 1968, 387 (1968) disclose the reaction of 2-vinylpyridine with an acetylenedicarboxylic acid ester to form the following compound:

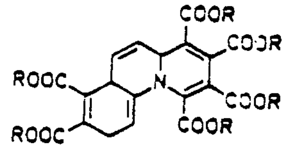

Gundermann et al., Justus Liebigs Ann. Chem., 10, 1873-1888 (1976) discloses 7- and 8-amino substituted naphtho[2,3-g]phthalazine-1,4(2H,3H)-diones having chemiluminescent quantum yields 2 to 3 times greater than luminol in an aprotic oxidation system. Specifically, 2,3-dihydro-naphtho[2,3-f]-

phthalazine-1,4-dione is described.

Sauer, Angew. Chem. Internat. Edit., 5, 211-223 (1966), Reed et al., The Journal of Organic Chemistry, 34(7), 2188-2191 (1969) and Sandermann et al., Tetrahedron Letters, No. 19, 1267-1268 (1963) also describe variations of the Diels-Alder reaction, including reactions employing acetylenes.

## Summary of the Invention

A process according to the present invention for synthesizing a cyclic hydrazide of a cyclic compound involves adding an $\alpha,\Omega$-acetylenedicarboxylic acid ester to an ethylene substituent of a cyclic compound to form a cyclic dicarboxylate of the cyclic compound, and reacting the cyclic dicarboxylate with hydrazine to form a cyclic hydrazide.

A conjugate according to the present invention may be formed by covalently coupling an antibody, a hapten, an antigen or a polynucleotide (e.g., DNA or RNA) to a chemiluminescent compound according to the present invention, and a method for performing a chemiluminescent assay comprises the step of exposing a sample to be tested to the conjugate in order to detect the presence of a substance specifically reactive with the conjugate, e.g., a specific antigen, a specific antibody or a complementary polynucleotide (i.e., a polynucleotide which forms sequence-specific hydrogen bonds with the polynucleotide conjugate according to the present invention).

A phenanthrene cyclic hydrazide according to the present invention is described by the formula

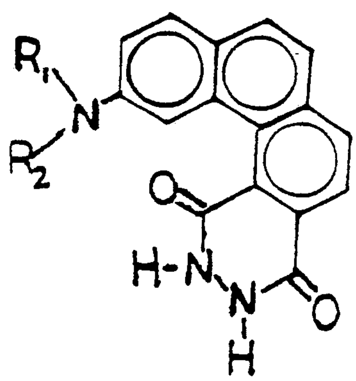

selected from the group consisting of compounds wherein:

$R_1$ and $R_2$ are independently -H or $-CH_3$ or $-CH_2CH_3$;

$R_1$ is -H, $R_2$ is $-(CH_2)_n\ SO_3H$ and n is 3 or 4;

$R_1$ and $R_2$ are $-(CH_2)_n\ SO_3H$ and n is 3 or 4; and

$R_1$ is $-CH_3$ or $-CH_2CH_3$ or $-(CH_2)_nSO_3H$ and n is 3 or 4; and $R_2$ is $-(CH_2)_n-NH-\overset{O}{C}-L$ and n is 2 to 8 wherein L is a specifically bindable ligand such as an antigen, a protein, a polypeptide, as hapten, an antibody, a hormone, a vitamin, a drug or a nucleic acid probe. In addition, this phenanthrene cyclic hydrazide may be used in an assay of the sort described in United States Patent No. 4,598,044.

## Brief Description of the Drawings

FIG. 1 is a flowchart for synthesis according to the prior art of a naphthalene derivative;

FIG. 2 is a flowchart for synthesis of a phenanthrene derivative according to the present invention; and

FIG. 3 is a flowchart for synthesis according to the present invention of the naphthalene derivative of FIG. 1.

## Detailed Description

Phenanthrene cyclic hydrazide and its derivatives are chemiluminescent compounds which have longer emmission wavelengths than most of the known cyclic hydrazides. Phenanthrene cyclic hydrazides may be used as labels in chemiluminescent immunoassays. The process according to present invention for synthesis of novel phenanthrene cyclic hydrazides and other known cyclic hydrazides is easier, and produces larger quantities of cyclic hydrazide in less time than other procedures. For example, Gundermann et al. disclosed the synthesis of 7- and 8- amino:substituted naphtho[2, 3-g]pthalazine-1,4-(2H, 4H)-diones in 8 steps [q.v. Justus Liebig's Ann. Chem., 10, 1873-1888 (1976)], but not of the phenanthrene cyclic hydrazides, which are described in this application.

Gundermann et al., Justus Liebig's Ann. Chem., 684, 127 (1965), and U.S. Patent No. 4,226,992 disclose a procedure for synthesizing an amino-functionalized napthalene 1,2-dicarboxylic acid hydrazide (compound VI, a compound described in Example 6, infra) in 12 steps, as illustrated in FIG. 1. A synthetic scheme according to the present invention results in the synthesis of compound VI and its isomers in larger quantities in much less time and also reduces the number of synthetic steps from 12 to 5, as illustrated in FIG. 3. Finally, the reaction of dialkyl derivatives of compound III or VIII, compounds described in Examples 3 and 8, with hydrazine produces the amino derivatives of cyclic hydrazide compounds (compounds VI and XI) as described in Examples 6 and 11.

Although other techniques may be employed to label antibodies, the NHS activation method is presently preferred. Other materials which function well according to the present invention include polyclonal antibodies, monoclonal antibodies, Fab antibody fragments, all of which are hereinafter included in the general term "antibody," haptens, antigens, nucleic acid probes, and binding proteins (for example, folate binding protein and intrinsic factor which binds vitamin $B_{12}$).

The present invention is more specifically described in the following Examples. Examples 1-6 are a description of the synthesis of an amino-functionalized phenanthrene cyclic hydrazide according to the present invention and as illustrated in FIG. 2. Set forth in Examples 7-11 is the synthesis of an amino-functionalized napthalene 1,2-dicarboxylic acid hydrazide according to the present invention and as illustrated in FIG. 3. Example 12 is a description of the preparation of another amino-functionalized phenanthrene cyclic hydrazide according to the present invention. Example 13 is a description of the preparation of an active ester of a compound according to the present invention, Examples 14 and 15 involve conjugation of the activated ester with an antibody. Example 16 is a description of a CLIA according to the present invention.

### Example 1

(Compound I) was prepared as follows. A solution of 10.0 of 2-vinylnaphthalene (Aldrich Chemical Co., Milwaukee, Wisconsin) and 9.7 g of dimethylacetylene dicarboxylate (Aldrich Chemical Co., Milwaukee, Wisconsin) in 200 ml of nitrobenzene was refluxed for 16 hours. The mixture was distilled under high vacuum to remove nitrobenzene and unreacted starting materials. the brown residue was chromatographed on a column of alumina oxide (neutral) eluted with benzene. A fast running yellow solution was combined and evaporated to give 6 g of phenathrene-1,2-dicarboxylate [compound (I)]. The mass spectrum of the compound was determined to have the following characteristics. M/e: 294 [$M^+$]; and 263 [$M^+$ minus O-$CH_3$], are peaks, such as the base peak ("$M^+$") in the mass spectrum at a specific location (i.e., at an M/e).

### Example 2

Next, 4.2 g (14.3 mmol) of compound (I) was dissolved in a large excess of trifluroacetic anhydride, the solution was cooled to 0°C by an ice bath. A solution of concentrated $HNO_3$ (17.1 mmol) was added dropwise by syringe. The reaction mixture was maintained at 0°C for 6 hours, then stirred overnight at room temperature. Removal of the solvent left a brown oil. This crude oil was chromatographed on a column of silica gel (E. Merck, Darmstadt, West Germany) eluting with $CHCl_3$, the yellow fraction was collected and evaporated to give 9-nitrophenanthrene-1,2-dicarboxylate [compound (II)] In the mass spectrum of the compound, M/e = 339 [$M^+$].

Example 3

Compound (II) was dissolved in absolute ethanol, 100 mg of catalyst of 10% Pd on carbon was added. The mixture was hydrogenated (at an initial pressure of 2 atmospheres) at room temperature for about 6 hours. Next, the resulting solution was filtered to remove the catalyst. Evaporation of the solvent gave 9-aminophenanthrene-1,2-dicarboxylate [compound (III)]. In the mass spectrum of the compound, M/e = 309 $[M^+]$.

Example 4

In a preparation following the procedure of Gundermann et al., Justus Liebig's Annalen der Chemie, 684, 127 (1965), a solution of 5 g of (16 mmol) of compound (III) and 4.6 g of N-(4-bromobutyl) phthalimide (Aldrich Chemical Co., Milwaukee, Wisconsin) in 500 ml of 2,2,2 trifluoroethenol was refluxed under $N_2(g)$ for 24 hours. Evaporation of solvent gave a dark red residue. This dark red residue was chromatographed on a column of silica gel (E. Merck, Darmstadt, West Germany) and eluted with a 19:1 (v/v) mixture of benzene and methanol. A fast moving red band was combined and evaporated to give 9-[4-(phthalimido)-butyl] aminophenanthrene-1,2-dicarboxylate [compound (IV)], the mass spectrum of which had the following characteristic: M/e = 510 $[M^+]$.

Example 5

A solution of compound (IV) and 10 ml diethyl sulfate was heated at 130°C for 24 hours under $N_2$. The resulting dark red solution was poured into a cold saturated, aqueous solution of sodium bicarbonate solution. This mixture was extracted 3 times with 200 ml of $CHCl_3$. The $CHCl_3$ extracts were combined, dried, and evaporated to give a red oil. The oil was chromatographed on a column of silica gel eluted with a 20:1 (v/v) mixture of benzene and methanol. The fast moving bands were combined and evaporated to give 9-[N-ethyl-N-(4-phthalimido)butyl]aminophenathrene-1,2-dicarboxylate [compound (V)], the mass spectrum of which, was characterized by: M/e = 538 $[M^+]$.

Example 6

One gram of compound (V), 5 ml of hydrazine, and 20 ml of ethanol were refluxed overnight. When cool, the reaction mixture was evaporated to dryness, the solid residue was dried overnight under high vacuum. The residue was chromatographed on a column of silica gel (E. Merck, Darmstadt, West Germany) eluted with a 7:3 (v/v) mixture of ethanol and 1 M triethylamine bicarbonate (pH 7.3). The major fractions were collected and evaporated to give an orange-yellow solid [compound (VI)]. Chemiluminescence was measured on a photon-counting luminometer. Light output was recorded as total photon counts, from which the efficiency of each compound was calculated as counts/mole. These are relative numbers, since the efficiency of the photon counting is instrument-dependent. The emission wavelength of this phenanthrene cyclic hydrizide derivatives extended beyond 600 nm.

One mg of a chemiluminescent compound to be tested was dissolved in 1 ml of DMF (Aldrich Chemical Co., Milwaukee, Wisconsin) in a cuvet. This stock solution was further diluted with 0.1 M PBS (pH 7.0) to approximately $10^{-8}$ M to $10^{-9}$ M in concentration, and an aliquot of 10 µl of chemiluminescent compound, 10 µl of catalyst,[i.e., microperoxidase at a concentration of approximately $10^{-13}$ M (Sigma Chemical Co., St. Louis, Missouri] or 0.3 mg/ml of Vanadium IV catalyst ("$V^{IV}$") or other metal ions (Aldrich Chemical Co., Milwaukee, Wisconsin) was mixed]. The cuvet was inserted into a photon counting luminometer, and 100 µl of 3% $H_2O_2$ in 0.25 N NaOH solution was injected into the reaction cuvet. The light emitted was measured for a period of 2 seconds immediately after injection.

The efficiency of chemiluminescent compounds depends on pH, solvents, and catalyst concentration. The efficiency of such compounds usually in a range of $10^{17}$ to $10^{19}$ counts/mole.

Example 7

A solution of 15 g of 4-nitrostyrene (Fairfield Chem. Co., Blythewood, South Carolina) and 14.29 g of dimethylacetylene dicarboxylate in 200 ml of nitrobenzene was refluxed for 24 hours. The excess of solvent was removed by high vacuum distillation. The resulting dark residue was chromatographed on a column of alumina oxides (neutral) and eluted with benzene. The eluted yellow solutions were combined and evaporated to give 6 g of 95% pure 7-nitronaphthelene-1,2-dicarboxylate [compound (VII)]. The mass spectrum of this compound was characterized by: M/e = 289 [$M^+$].

Example 8

Six grams of compound (VII) were dissolved in 250 ml of absolute ethanol, 100 mg of 10% Pd on carbon was added. The resulting solution was hydrogenated (at an initial pressure of 2 atomosphere) for about 6 hours until uptake of $H_2$ stopped. The highly fluorescent resulting solution was filtered to remove carbon. Evaporation of the solvent gave 7-aminonaphthalene-1,2-dicarboxylate [compound (VIII)], the mass spectrum of which was characterized by: M/e = 259 [$M^+$].

Example 9

A solution of 1.7 g of compound VIII, 1.85 g of N-(4-bromobutyl) phthalimide (Aldrich Chemical Co., Milwaukee, Wisconsin) and 20 ml of 2,2,2 trifluroethanol was refluxed under $N_2$ for 24 hours. Evaporation of solvent gave a dark residue. A solution of water/ether (250 ml/500 ml) was added, and the ether layer was separated, dried and evaporated to dryness to give a red oil. The red oil was chromatographed on a column of silica gel (E. Merck, Darmstadt, West Germany) and eluted with a 18:1 (v/v) mixture of benzene and methanol. The resulting yellow fractions were combined and evaporated to give a red oil. The oil was (7[4-N-(phthalimido)butyl] aminonaphthalene-1,2-dicarboxylate [compound (IX)], the mass spectrum of which was characterized by: M/e = 460 [$M^+$].

Example 10

Next, compound (IX) was added to a large excess of iodoethane. Evaporation of excess iodoethane gave a red oil. The crude red oil was chromatographed on a column of silica gel (E. Merck, Darmstadt, West Germany) and eluted with a mixture of 19/1 (V/V) benzene and methanol. The resulting yellow fractions were combined and evaporated to give 7-(N-ethyl-N-[4-(N-phthalimido)butyl] amino) naphthalene -1,2-dicarboxylate [compound (X)], the mass spectrum of which was characterized by: M/e = 488 [$M^+$].

Example 11

To four grams of compound (X) in a 250 ml round bottom flask was added 10 ml of $NH_2NH_2$ and 100 ml of ethanol. The resulting solution was refluxed overnight. Upon cooling, the solution was evaporated to dryness to give a yellow solid. The crude solid was recrystallized with dry pyridine and the yellow solid was collected to give 7-[N-4-aminobutyl-N-ethyl] aminonaphthalene-1,2-dicarboxylic acid hydrazide [compound (XI)].

Example 12

Five hundred milligrams of compound III (1.6 mmol) and 0.79 g of 1,3 propane sultone) (Aldrich Chem.

Co., Milwaukee, Wisconsin) were heated to 130° C for 4 hours after which the reaction mixture was allowed to cool to room temperature. The residue was washed with benzene to remove excess of 1,3-propane sultone, and then a resulting amide solid was evaporated to dryness to remove any excess solvent. To the resulting material was added 2 ml of $NH_2NH_2$ and 10 ml of ethanol. The mixture was refluxed overnight. After cooling, the solution was evaporated to dryness to give 9-(dipropane sulfonic acid) aminophenathrane -1,2- dicarboxylic acid hydrazide, the mass spectrum of which was characterized by: M/e = 522 [$M^+H$].

## Example 13

One hundred milligrams of compound VI or XI and one equimolar of succinic anhydride (Aldrich Chemical Co., Milwaukee, Wisconsin) was stirred at room temperature in dry pyridine (Aldrich Chemical Co., Milwaukee, Wisconsin). The reaction was monitored by thin layer chromatography (TLC) ($CHCl_3$:MeOH, 2:1). A yellow band with an $R_f$ value of 0.55 was isolated by preparative TLC to afford 45 mg of a yellow solid as hemisuccinate of compound VI or XI. One milligram of the hemisuccinate was redissolved in dry DMF (Aldrich Chemical Co., Milwaukee, Wisconsin), added 1 equimolar of dicyclohexyl carbodimide [-("DCC") Aldrich Chemical Co., Milwaukee, Wisconsin], and 1 equimolar of N-hydroxysuccimide ("NHS", Aldrich Chemical Co., Milwaukee, Wisconsin). The mixture was stirred at room temperature overnight to allow the formation of an active ester.

## Example 14

One milligram of rabbit IgG (Sigma Chemical Co., St. Louis, Missouri) was dissolved in 0.1 M sodium phosphate buffer (pH 7.0) containing 1% Tween 80®. Thirty-Five microliters of an activated ester from Example 13 solution was added with constant stirring. The reaction proceded for 6 hours. The mixture was chromatographed over Sephadex® G-25 (10 cm X 0.75 cm) as available from Pharmacia, Piscatway, New Jersey, and eluted with 0.1 M PBS (pH = 7.0). The labelled conjugate eluted as weakly yellowish fluorescent band. The labelled protein may be further purified by HPLC using a Bio-Sil® TSK-250 column. (BioRad, Richmond, California). The resulting conjugate contained about 4 to 6 labels/protein, as determined from the ratio of the absorbance at 280 nm to the absorbance at 320 nm.

## Example 15

A solution of an activated ester from Example 13 was mixed with mouse monoclonal CEA-antibody in 0.1 M sodium phosphate buffer (pH 7.0) in a molar ratio of 30:1 at 2-8° C for about 6 hours.

The conjugate was then dialysed against PBS buffer, pH 7.0, until the absorbance of the dialysate indicated that no free label was present. A UV spectral analysis indicated between 4 to 6 labels/antibody (as determined from a ratio of absorbance as in Example 14).

## Example 16

A cyclic hydrazide-labelled CEA conjugate was diluted to 250 ng/ml in PBS, 0.01% Tween®, pH 7.0. Using a CEA kit purchased from Abbott Laboratories, Abbott Park, Illinois, an assay was performed as follows.

Fifty microliters of a specimen diluent 100 µl of conjugate and an Abbott one-quarter inch bead were combined in a well. The combination was incubated at 37° C for 1 hour, and washed four times with distilled water. The washed bead was transferred into TDX® cuvet, and 100 µl of 0.3 mg/ml of $V^{IV}$ catalyst was added. After addition of 200 µl of 3% $H_2O_2$ in 0.25 N NaOH solution, the results were immediately read for 2 seconds on a photoluminometer.

The results demonstrated that the assay will quantitatively measure CEA in the clinically significant range of 0 ng/ml to 80 ng/ml.

It is understood that those skilled in the art will be enabled by the above specification to incorporate reactive functional groups for attaching the label to an analyte into compounds according to the present invention. It is also contemplated that compounds according to the present invention may be: used in labeling DNA probes; incorporated into an enzyme substrate wherein the product of the enzymatic reaction is the chemiluminescent compound; and incorporated into systems which involve energy transfer or fluorescent quenching.

It is expected that other variations and improvements will occur to those skilled in the art upon consideration of the present invention. For example, inasmuch as 2-vinylnaphthalene represents isomers (I) and (II) as follows,

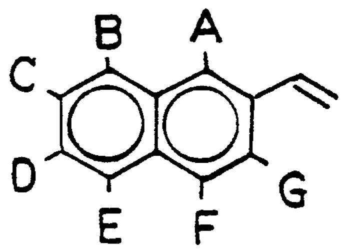

(I)

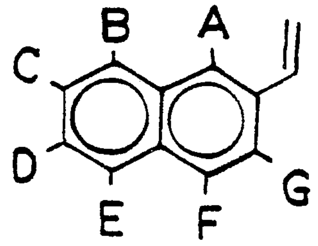

(II)

it is understood that anthrecene cyclic hydrazides and phenanthrene cyclic hydrazides may be manufactured according to the present invention and that they may have the respective formulas (III) and (IV):

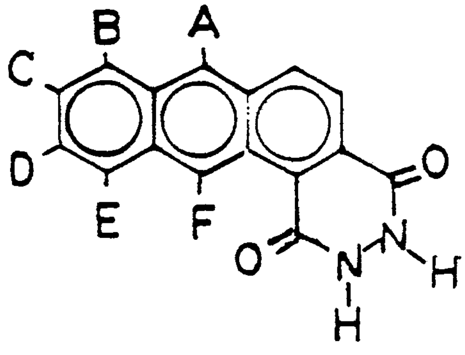

(III)

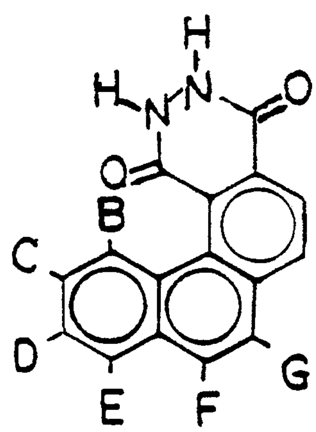

(IV)

wherein for all of (I), (II), (III) and (IV), one of B or C may be selected from the group including $-NO_2$, $-NH_2$, -OH, $-OCH_3$ and a halogen, while for (II) and (IV) one of F or G may be selected from this group and for (I) and (III) A may be selected from this group.

Therefore, it is intended that the present invention include all such variations and improvements as come within the scope of the invention as claimed.

## Claims

1. A process for synthesizing a cyclic hydrazide of a cyclic compound comprising the steps of:
adding an α,Ω-acetylenedicarboxylic acid ester to an ethylene substituent of a cyclic compound to form a cyclic dicarboxylate of the cyclic compound; and
reacting said cyclic dicarboxylate with hydrazine to form a chemiluminescent cyclic hydrazide.

2. A chemiluminescent phenanthrene cyclic hydrazide having the formula:

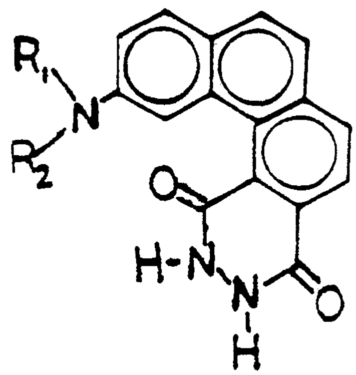

selected from the group consisting of compounds wherein:
$R_1$ and $R_2$ are independently $-H$ or $-CH_3$ or $-CH_2CH_3$;
$R_1$ is $-H$, $R_2$ is $-(CH_2)_n\ SO_3H$ and n is 3 or 4;
$R_1$ and $R_2$ are $-(CH_2)_n\ SO_3H$ and n is 3 or 4; and
$R_1$ is $-CH_3$ or $-CH_2CH_3$ or $-(CH_2)_nSO_3H$ and n is 3 or 4; and $R_2$ is
$-(CH_2)_n-NH-\overset{O}{C}-L$ and n is 2 to 8 wherein L is a specifically bindable ligand such as an antigen, a protein, a polypeptide, as hapten, an antibody, a hormone, a vitamin, a drug or a nucleic acid probe.

3. The chemiluminescent compound as recited in claim 2 wherein said chemiluminescent compound is identified by the formula 9-[N-ethyl-N-butyl] aminophenanthrene-1,2-dicarboxylic acid hydrazide.

4. The chemiluminescent compound as recited in claim 2 wherein said chemiluminescent compound is identified by the formula 9-(dipropane sulfonic acid)aminophenanthrene-1,2-dicarboxylic acid hydrazide.

5. A conjugate formed by an antibody conjugated to a chemiluminescent compound as recited in claim 2.

6. A method for performing a chemiluminescent immunoassay to test for the presence of a substance comprising the step of exposing a sample to a conjugate as recited in claim 5.

7. A conjugate formed by an antigen conjugated to a chemiluminescent compound as recited in claim 2.

8. A method for performing a chemiluminescent immunoassay to test for the presence of an antibody to an antigen as recited in claim 7 comprising the step of exposing a sample to a conjugate as recited in claim 7.

9. A conjugate formed by an antibody conjugated to a chemiluminescent compound as recited in claim 2.

10. A method for performing a chemiluminescent immunoassay to test for the presence of a substance comprising the step of exposing a sample to a conjugate as recited in claim 9.

11. A conjugate formed by an antigen conjugated to chemiluminescent compound as recited in claim 3.

12. A method for performing a chemiluminescent immunoassay to test for the presence of an antibody to the antigen as recited in claim 11 comprising the step of exposing a sample to a conjugate as recited in claim 11.

13. A conjugate formed by a polynucleotide conjugated to a chemiluminescent compound as recited in claim 2.

14. A method for performing a chemiluminescent assay comprising the step of exposing a sample to be tested to a polynucleotide conjugate as recited in claim 13 in order to detect the presence of a polynucleotide complementary to the conjugate.

15. A conjugate formed by a polynucleotide conjugated to a chemiluminescent compound as recited in claim 3.

16. A method for performing a chemiluminescent assay comprising the step of exposing a sample to be tested to a conjugate as recited in claim 3 in order to detect the presence of a polynucleotide complementary to the nucleic acid in the conjugate.

Neu eingereicht / Newly filed
Nouvellement déposé

FIG. 1 PRIOR ART

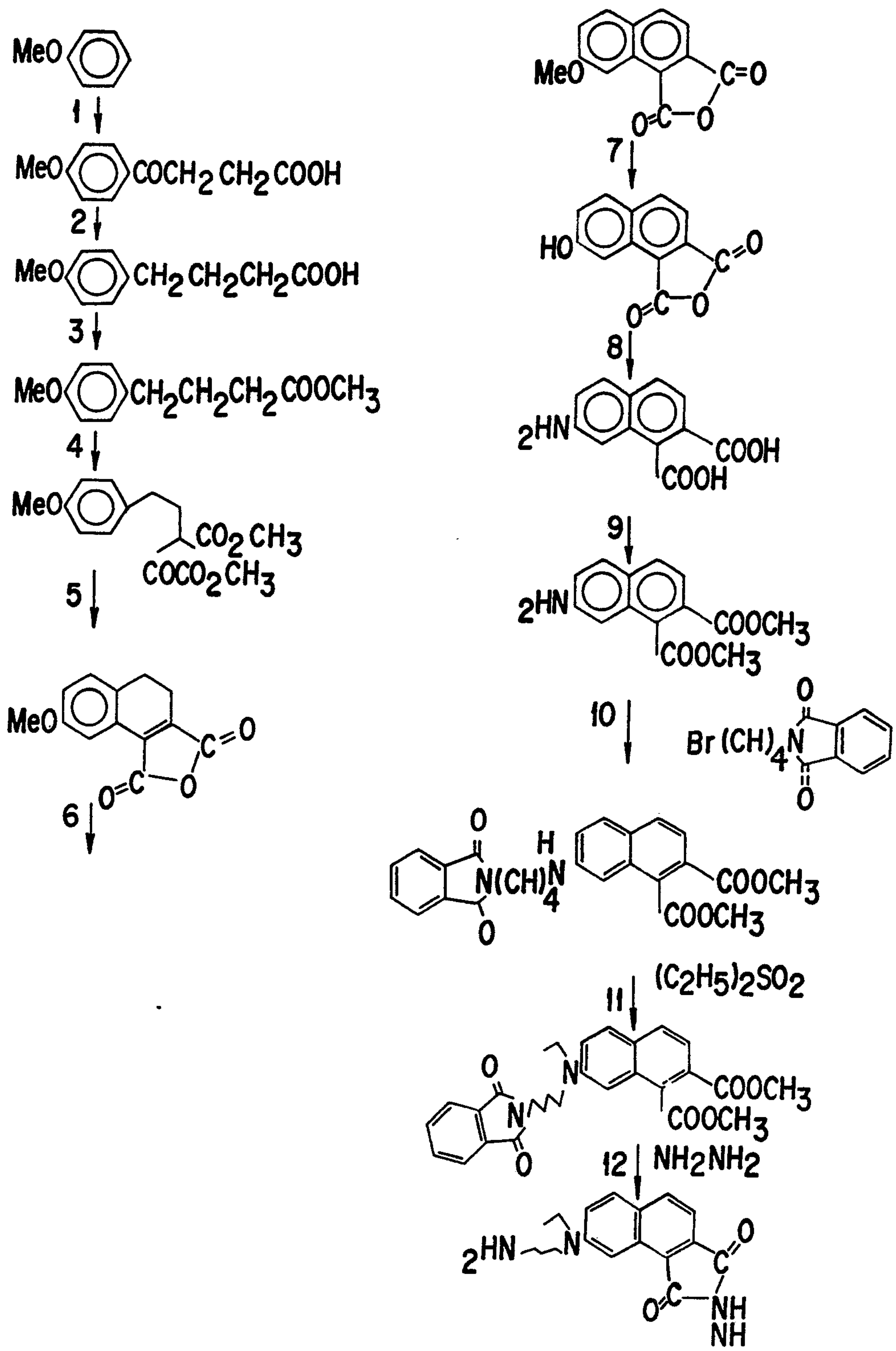

FIG.2

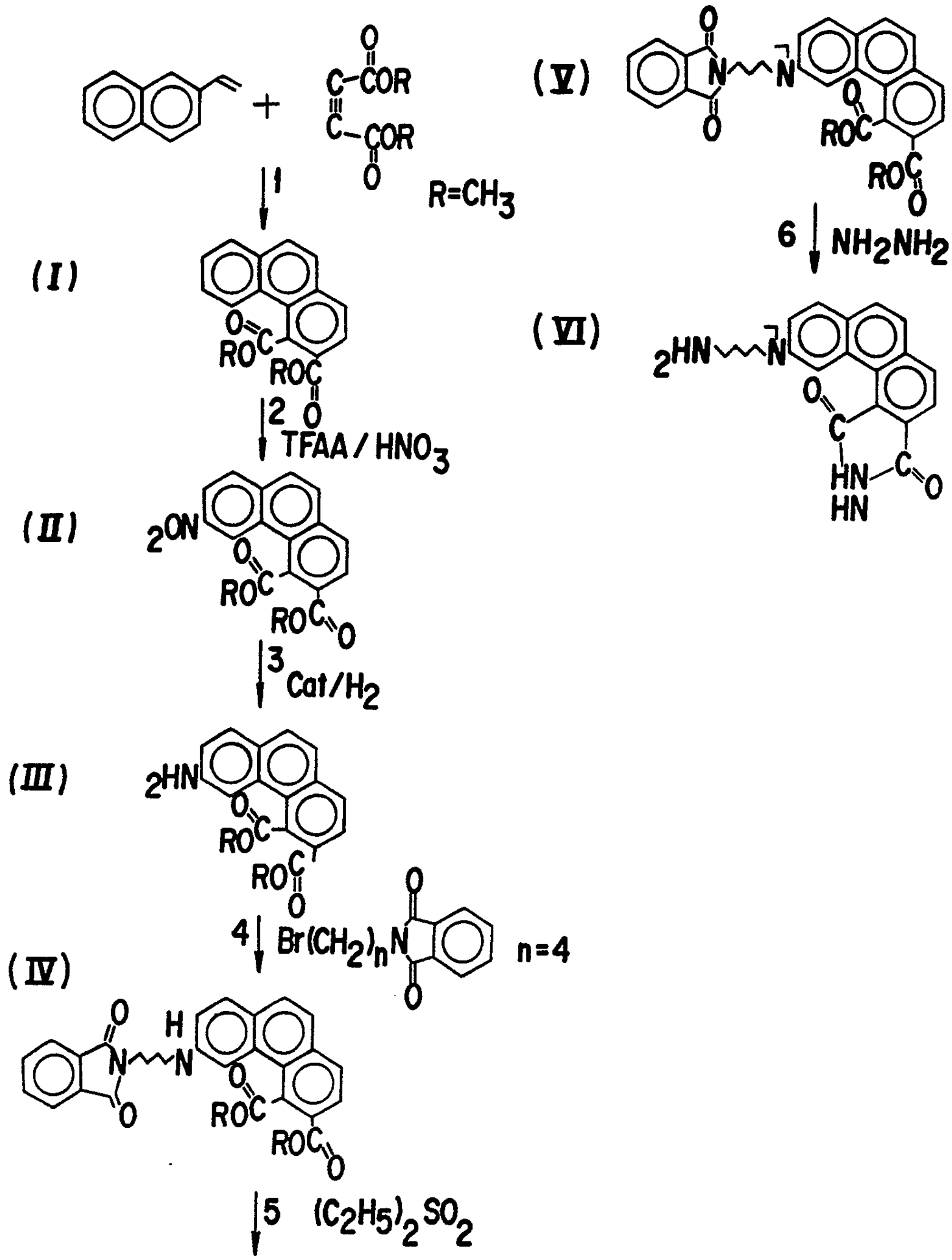


(I)

(II)

(III)

(IV)

(V)

(VI)

FIG. 3

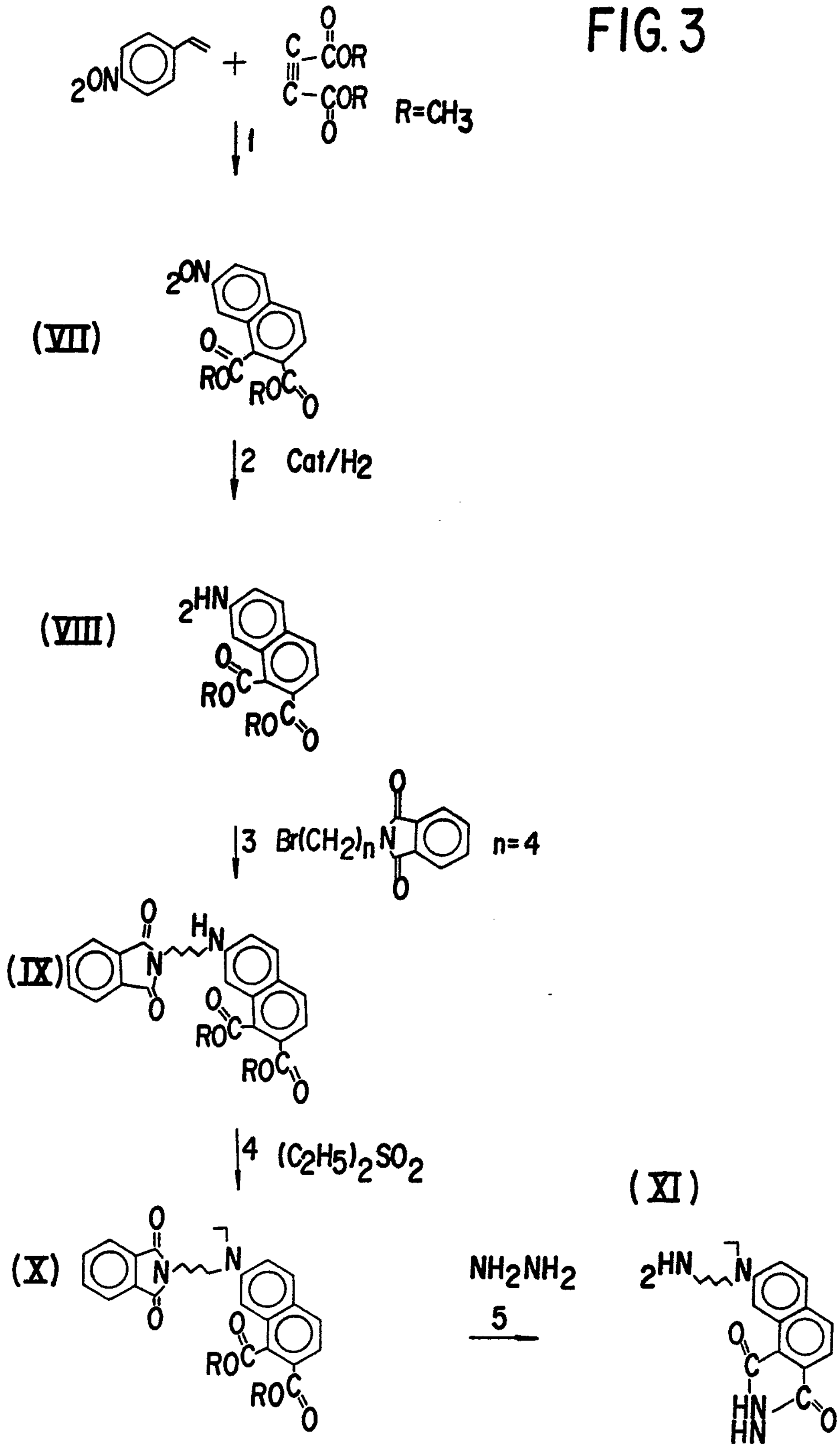


(VII)

(VIII)

(IX)

(X)

(XI)